# EUROPÄISCHE PATENTSCHRIFT

(11) **EP 0 345 475 B1**
(45) Veröffentlichungstag und Bekanntmachung des Hinweises auf die Patenterteilung: **21.04.1993**
(21) Anmeldenummer: 89108353.7
(22) Anmeldetag: 10.05.1989
(51) Int. Cl.: C07C 209/04, C07C 211/03, C11D 1/65

(54) **Verfahren zur Herstellung von quaternären Ammoniumsalzen langkettiger aliphatischer Carbonsäuren**
Process for the production of quaternary ammonium salts of long-chain aliphatic carboxylic acids
Procédé de production de sels d'ammonium quaternaires d'acides carboxyliques de chaîne aliphatique longue

(30) Priorität: 13.05.1988 DE 3816328
(43) Veröffentlichungstag der Anmeldung: 13.12.1989
(73) Patentinhaber: HOECHST AKTIENGESELLSCHAFT, 65926 Frankfurt am Main (DE)
(72) Erfinder: Wehle, Detlef, Dr., D-8261 Kastl/Obb. (DE); König, Franz, D-6000 Frankfurt am Main 71 (DE)

(56) Entgegenhaltungen:
- EP-A- 0 189 085
- EP-A- 0 269 949
- EP-A- 0 281 975
- EP-A- 0 291 074
- GB-A- 1 050 791
- US-A- 4 392 965
- CHEMICAL ABSTRACTS, Band 109, 1988, Seite 807, Zusammenfassung Nr. 140942c, Columbus, Ohio, US; & JP-A-63 119 214 (NIPPON CHEMI-CON CORP.) 23-05-1988
- CHEMICAL ABSTRACTS, Band 108, 1988, Seite 535, Zusammenfassung Nr. 194813j, Columbus, Ohio, US; & JP-A-63 24 080 (S. SHIMIZU et al.) 01-02-1988

## Beschreibung

Die Erfindung betrifft ein Verfahren zur Herstellung von quaternären Ammoniumsalzen langkettiger aliphatischer Carbonsäuren, die am Stickstoffatom drei kurzkettige und einen langkettigen aliphatischen Kohlenwasserstoffrest tragen.

Es ist aus IT-PS 1 153 530 bekannt, quaternäre Ammonium-Alkylcarbonate durch Umsetzung von tertiären Aminen, beispielsweise unter anderem Dimethylstearylamin mit Dialkylcarbonaten, beispielsweise Dimethylcarbonat, gegenbenenfalls in Gegenwart eines Katalysators und/oder in Gegenwart eines Lösungsmittels, herzustellen und nach Abtrennung des überschüssigen Dialkylcarbonates mit einer stöchiometrischen Menge einer Säure, wie Ameisen-, Essig-, Chlorwasserstoff-, Bromwasserstoff-, Iodwasserstoff- oder Schwefelsäure, bei 20 bis 25 °C weiter umzusetzen, wobei die quaternären Ammoniumsalze dieser Säuren erhalten werden. Über eine Umsetzung mit längerkettigen Carbonsäuren und die dabei anzuwendenden Bedingungen ist nichts ausgesagt.
Es sind ferner aus US-PS 3,223,718 quaternäre Ammoniumsalze längerkettiger Carbonsäuren, wie Stearinsäure, und ihr Einsatz als Wäscheweichmacher bekannt, die am Stickstoffatom neben einem oder zwei langkettigen Alkylresten eine -CH₂CH₂OH-Gruppe tragen.

Aus US-PS 4,392,965 sind unter anderem quaternäre Ammoniumsalze von Carbonsäuren mit 12 bis 22 C-Atomen als Wäscheweichmacher bekannt, die am Stickstoffatom zwei längerkettige Alkylreste aufweisen.

Weiterhin ist aus EP 7 135-B1 ein Fabrikationsgegenstand zum Weichmachen von Textilien in einem Wäschetrockner bekannt, der eine weichmachende Menge eines Textilweichmachungsmittels mit einem Schmelzpunkt von 35 bis 100 °C enthält und 5 bis 100 % eines tertiären Aminsalzes einer aliphatischen Carbonsäure mit 12 bis 22 C-Atomen, das am Stickstoffatom neben einem Wasserstoffatom mindestens eine aliphatische Gruppe mit 12 bis 22 C-Atomen enthält.

Die beschriebenen Ammonium- beziehungsweise Amin-Salze weisen mindestens einen der folgenden Nachteile auf: a) aufwendige Herstellung; b) mangelnde Stabilität gegen Hydrolyse bei der Lagerung für sich allein oder zusammen mit anderen Textilbehandlungsmitteln, wodurch die Viskosität und andere Eigenschaften als Textilweichmacher negativ beeinflußt werden und c) über den gesamten pH-Bereich nicht gleichmäßige kationische Ladung, wodurch in bestimmten Formulierungen die Wirksamkeit ungünstig beeinflußt wird.

Schließlich wird in GB-PS 1 235 107 ein Verfahren beschrieben zum Färben von Materialien, die ganz oder teilweise aus synthetischem Polyamid bestehen in Gegenwart von Jute, wobei neben dem Färbemittel unter anderem Cetyltrimethyl-ammonium-stearat verwendet wird.

Aufgabe der vorliegenden Erfindung ist es, ein Verfahren bereitzustellen, mit dem quaternäre Ammoniumsalze von längerkettigen Carbonsäuren, die am Stickstoffatom einen langkettigen und drei kurzkettige Alkylreste tragen, mit guten Ausbeuten ohne den Anfall von abwasserbelastenden Salzen erzeugt werden können. Eine weitere Aufgabe der Erfindung ist es, Verbindungen für Wäscheweichspülmittel zur Verfügung zu stellen, die die oben näher erläuterten Nachteile nicht aufweisen und insbesondere hydrolysestabil sind.

Das erfindungsgemäße Verfahren zur Herstellung von quaternären Ammoniumsalzen langkettiger aliphatischer Carbonsäuren, die am Stickstoffatom drei kurzkettige und einen langkettigen aliphatischen Kohlenwasserstoffrest tragen, ist dadurch gekennzeichnet, daß man
a) 1 mol von mindestens einer Verbindung der Formel 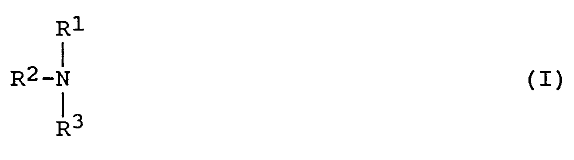 in der bedeuten
   R¹ einen geradkettigen oder verzweigten, aliphatischen Kohlenwasserstoffrest mit 8 bis 22 C-Atomen, der 1 bis 3 C=C-Doppelbindungen und/oder 1 bis 3 OH-Gruppen enthalten kann, und R² und R³, gleich oder verschieden, je einen Alkylrest mit 1 bis 4 C-Atomen,
   mit 0,5 bis 6 mol einer Verbindung der Formel

   R⁴OCOOCH₃ (II)

   in der R⁴ Methyl- oder Ethyl- bedeutet,
   bei 100 bis 200 °C unter einem Druck von 0,095 bis 1,5 MPa umsetzt,
b) von der im Schritt a) erhaltenen Reaktionsmischung die Hauptmenge der nicht umgesetzten Verbindung der Formel (II) gegebenenfalls abtrennt,
c) der im Schritt a) oder, sofern ausgeführt, im Schritt b) erhaltenen Reaktionsmischung je 1 mol der eingesetzten Verbindung der Formel (I) 1 bis 2 mol von mindestens einer Carbonsäure der Formel

   R⁵COOH (III)

   worin R⁵ einen geradkettigen oder verzweigten, aliphatischen Kohlenwasserstoffrest mit 8 bis 40 C-Atomen, der 1 oder 2 COOH-Gruppen, 1 bis 3 C=C-Doppelbindungen, 1 bis 3 CHOH-Gruppen sowie einen Ring enthalten kann,
   bedeutet, bei einer Temperatur von 40 bis 140 °C zusetzt und diese Temperatur aufrechterhält, bis keine CO₂-Entwicklung mehr auftritt, und
d) die restliche Menge an CO₂ und den im Schritt c) ebenfalls gebildeten Alkohol R⁴OH von der im Schritt c) erhaltenen Reaktionsmischung abdestilliert, wobei das gewünschte quaternäre Ammoniumsalz der Formel 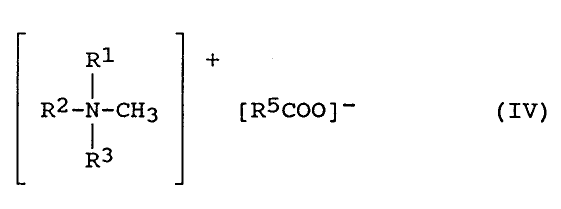 erhalten wird.

Wegen der günstigen Eigenschaften der daraus erzeugten Verbindungen der Formel (IV) werden bevorzugt als Verbindungen der Formel (I) solche eingesetzt, in denen R¹ einen geradkettigen oder verzweigten aliphatischen Kohlenwasserstoffrest mit 12 bis 20 C-Atomen bedeutet, der 1 bis 3 C=C-Doppelbindungen und/oder 1 bis 3 OH-Gruppen enthalten kann. Insbesondere werden solche Verbindungen der Formel (I) verwendet, bei denen R¹ einen geradkettigen oder verzweigten aliphatischen Kohlenwasserstoffrest mit 14 bis 18 C-Atomen bedeutet, der eine C=C-Doppelbindung und/oder eine OH-Gruppe enthalten kann. Die Reste R² und R³ in der Verbindung der Formel (I) bedeuten vorzugsweise Methyl- oder Ethyl, wobei sie unter sich gleich oder verschieden sein können. Für das erfindungsgemäße Verfahren können auch mehrere Verbindungen der Formel (I) eingesetzt werden.

Als Verbindungen der Formel (II) wird wegen der leichten Beschaffbarkeit und guten Reaktionsfähigkeit bevorzugt Dimethylcarbonat eingesetzt.

Je 1 mol einer oder mehrerer Verbindungen der Formel (I) werden 0,5 bis 6 mol der Verbindung der Formel (II) eingesetzt. Prinzipiell können auch mehr als 6 mol der Verbindung (II) verwendet werden, jedoch würde dies einen zusätzlichen Aufwand bedeuten, der in keinem Verhältnis zu der zusätzlich erzielten Wirkung steht. Vorzugsweise werden von der Verbindung der Formel (II) 0,7 bis 5 und insbesondere 1 bis 3 mol je 1 mol der Verbindung der Formel (I) eingesetzt.

Sofern von der Verbindung der Formel (II) weniger als 1 mol je 1 mol der Verbindung der Formel (I) verwendet wird, bleibt ein Teil von letzterer Verbindung unverändert im Reaktionsgemisch enthalten. Dieser Teil bildet bei der anschließenden Umsetzung mit einer Carbonsäure der Formel (III) ein Aminsalz der Formel 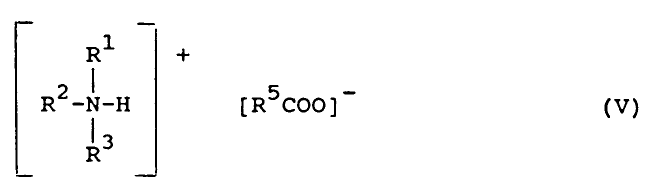
so daß eine Mischung von Verbindungen der Formeln (IV) und (V) erhalten wird. Das gleiche wird erreicht, wenn zwar von der Verbindung der Formel (II) 1 mol oder mehr je 1 mol der Verbindung der Formel (I) verwendet, jedoch die Umsetzung der beiden Verbindungen vorzeitig, beispielsweise durch Abdestillieren der Verbindung der Formel (II) beendet wird. Allerdings muß beim letzteren Verfahren ein Teil der Verbindung der Formel (II) abgetrennt werden, was bei dem zuvor beschriebenen Verfahren nicht erforderlich ist. Die erzeugten Mischungen der Verbindungen der Formeln (IV) und (V) können für manche Anwendungsgebiete gleich gut oder sogar mit Vorteil gegenüber den Verbindungen, die ausschließlich der Formel (IV) entsprechen, eingesetzt werden.

Die Reaktion der Verbindungen der Formeln (I) und (II) wird bei einer Temperatur von 100 bis 200 °C durchgeführt. Unter 100 °C verläuft die Reaktion im allgemeinen unnötig langsam, oberhalb 200 °C treten in zunehmendem Maße unerwünschte Nebenreaktionen auf. Vorzugsweise wird im Temperaturbereich von 120 bis 160 °C gearbeitet. Der Druck ist für die Reaktion nicht kritisch, zweckmäßig wird sie unter normalem Atmosphärendruck oder, wenn dieser höher liegt, unter dem autogenen Druck der Reaktionsmischung durchgeführt. Der angewendete Druckbereich beträgt etwa 0,095 bis 1,5, vorzugsweise 0,095 bis 0,5 MPa.

Die Dauer der Reaktion zwischen den Verbindungen der Formel (I) und (II) hängt von der gewählten Reaktionstemperatur, den Reaktionspartnern und den Mengenverhältnissen ab. Im allgemeinen ist die Reaktion innerhalb 1 bis 12 Stunden beendet. Sofern ein molarer Überschuß an Verbindungen der Formel (II) verwendet wurde, wird die Hauptmenge der bei der Reaktion unverändert gebliebenen Verbindung beziehungsweise Verbindungen der Formel (II) abgetrennt. Dies kann auf verschiedenen Wegen geschehen, beispielsweise durch Destillieren, wenn erforderlich unter vermindertem Druck, bei Temperaturen von 80 bis 100 °C, wobei zur Erleichterung der Abtrennung organische inerte Flüssigkeiten, die mit der oder den abzutrennenden Verbindungen der Formel (II) ein azeotropes Gemisch bilden, zugesetzt werden können. Geeignete Verbindungen sind beispielsweise Alkanole, wie n-Propylalkohol, sec-Butylalkohol oder Isobutylalkohol, insbesondere Methanol, Ethanol, Isopropanol sowie auch deren Mischungen.

In einer Ausführungsform des erfindungsgemäßen Verfahrens können, nach Beendigung der Reaktion, zwischen den Verbindungen der Formeln (I) und (II), und gegebenenfalls Abtrennung eines Teils der nichtumgesetzten Verbindung beziehungsweise Verbindungen der Formel (II), der Reaktionsmischung je 1 mol der in dieser Mischung noch verbliebenen Verbindung beziehungsweise Verbindungen der Formel (II) 1 bis 10 mol Wasser zugesetzt werden. Neben dem wasserzusatz können organische Flüssigkeiten, beispielsweise Methanol, Ethanol oder Isopropanol eingesetzt werden, wobei die Verbindung beziehungsweise die Verbindungen der Formel (II) als tertiäre Azeotrope flüchtig sind. Prinzipiell können auch mehr als 10 mol Wasser verwendet werden, jedoch bringt dies im allgemeinen keinen zusätzlichen Effekt.

Nach Abtrennung der Hauptmenge der nicht-umgesetzten Verbindung beziehungsweise Verbindungen der Formel (II) wird die Temperatur der Reaktionsmischung auf 40 bis 140 °C eingestellt und je 1 mol der eingesetzten Verbindung beziehungsweise Verbindungen der Formel (I) 1 bis 2, vorzugsweise 1 bis 1,2 mol von mindestens einer Carbonsäure der Formel R⁵ COOH (III) zugesetzt. In der genannten Formel hat R⁵ die weiter oben angegebene Bedeutung. Vorzugsweise wird mindestens eine Carbonsäure der Formel (III) eingesetzt, in der R⁵ einen geradkettigen oder verzweigten aliphatischen Kohlenwasserstoffrest mit 8 bis 22 C-Atomen bedeutet, der 1 bis 3 C=C-Doppelbindungen und/oder 1 bis 3 OH-Gruppen enthalten kann. Besonders bevorzugt ist R⁵, ein geradkettiger oder verzweigter aliphatischer Kohlenwasserstoffrest mit 14 bis 20 C-Atomen. Geeignete Carbonsäuren sind beispielsweise Caprylsäure, Pelargonsäure, Caprinsäure, Undecansäure, Laurinsäure, Myristinsäure, Pentadecansäure, Palmitinsäure, Stearinsäure, Arachinsäure (Eicosansäure), Behensäure, Lauroleinsäure, Myristoleinsäure, Palmitoleinsäure, Ölsäure, Erucasäure, Linolsäure, Eicosadiensäure, Linolensäure, Elaeostearinsäure, Calendulasäure, Petroselinsäure, Hydroxystearinsäure, Ricinelaidinsäure, Ricinolsäure, Sebacinsäure, Octadecandicarbonsäure, dimerisierte oder trimerisierte ungesättigte Fettsäuren, wie dimerisierte Linolsäure, oder die Produkte Pripol® 1017 der Firma Unichema, Emmerich, sowie Diacid® 1550 der Firma Westvaco. Es können auch Mischungen verschiedener Carbonsäuren verwendet werden.

Wird vor oder nach Zusatz der Verbindung beziehungsweise Verbindungen der Formel (III) die Temperatur der Reaktionsmischung auf weniger als 40 °C eingestellt, so wird eine unnötig langsame, wenig vollständige Reaktion beobachtet, auch erfordern so niedrige Temperaturen oft die Anwendung unnötig hoher Lösungsmittel-Mengen, um die Reaktion in flüssiger Phase durchführen zu können. Oberhalb 140 °C treten unerwünschte Nebenreaktionen sowie in zunehmendem Maße Zersetzung der erzeugten Verbindung beziehungsweise der Verbindungen der Formel (IV) ein. Vorzugsweise wird die Temperatur der Reaktionsmischung auf 60 bis 120 °C und insbesondere auf 80 bis 100 °C eingestellt. Der herrschende Druck ist nicht kritisch. Man arbeitet zweckmäßig bei Atmosphärendruck beziehungsweise dem autogenen Druck der Reaktionsmischung.

In einer bevorzugten Ausführungsform der Erfindung wird ohne Zusatz von Lösungsmitteln gearbeitet. Es kann jedoch in bestimmten Fällen nützlich sein, Lösungsmittel zu verwenden, insbesondere dann, wenn bei niedrigen Reaktionstemperaturen im Bereich von etwa 40 bis 70 °C gearbeitet werden soll, in dem die eingesetzte Carbonsäure nicht oder nur teilweise geschmolzen ist beziehungsweise wenn die Viskosität der Reaktionsmischung so hoch ist, daß mit üblichen Methoden, wie zum Beispiel Rühren, nur eine ungenügende Durchmischung erfolgt. Geeignete Lösungsmittel sind beispielsweise n-Propanol, Dialkylether mit 2 bis 6 C-Atomen in den Alkylresten, Ethylenglykol und seine höheren Homologen, wie Diethylenglykol, Triethylenglykol sowie deren Monoalkylether mit 1 bis 3 C-Atomen in der Alkylgruppe und insbesondere Methanol, Ethanol, Isopropanol, n-Butanol, iso-Butanol sowie Dialkylglykolether mit 1 bis 3 C-Atomen in den Alkylgruppen. Es können ein oder mehrere Lösungsmittel eingesetzt werden, wobei deren Menge zweckmäßig so bemessen wird, daß die Reaktionsmischung homogen flüssig und nicht zu hoch viskos ist.

Wird bei höheren Reaktionstemperaturen, beispielsweise uber 60 °C, gearbeitet, ist es häufig empfehlenswert, die Verbindung beziehungsweise die Verbindungen der Formel (III), sofern sie nicht schon flüssig sind, vor der Zugabe über ihren Schmelzpunkt zu erwärmen.

Nach Zugabe der Verbindung beziehungsweise der Verbindungen der Formel (III) zur Reaktionsmischung aus den Verbindungen der Formeln (I) und (II) entwickelt sich CO₂, ferner wird, insbesondere bei höheren Reaktionstemperaturen, auch die entstandene Verbindung der Formel R⁴OH dampfförmig abdestilliert. Das Ende der Reaktion ist im allgemeinen dadurch zu erkennen, daß keine Gasentwicklung mehr auftritt. Die Reaktionszeit ist, je nach gewählter Reaktionstemperatur und den eingesetzten Reaktionspartnern, verschieden und liegt im allgemeinen zwischen 0,1 und 5 Stunden.

Während und nach Beendigung der Reaktion, die bei Zugabe der Verbindung beziehungsweise der Verbindungen der Formel (III) eintritt, können, wenn erforderlich, Reste von CO₂ und die entstandene Verbindung der Formel R⁴OH sowie eventuell verwendete Lösungsmittel, unter vermindertem Druck, beispielsweise 0,5 bis 80 kPa bei Temperaturen von 60 bis 110 °C, vorzugsweise 80 bis 100 °C, abdestilliert werden. Eine Wiedergewinnung der Verbindung R⁴OH durch Kondensation ist zweckmäßig. Während dieser Destillation, wie auch während der weiter oben beschriebenen Abtrennung der überschüssigen Verbindung beziehungsweise Verbindungen der Formeln (II) in Dampfform aus der Reaktionsmischung, kann es zweckmäßig sein, unter einem inerten Schutzgas, beispielsweise Stickstoff oder Argon, zu arbeiten.

Die erfindungsgemäß hergestellte Verbindung der Formel (IV) verbleibt als Rückstand und kann, wenn erforderlich, in einer weiteren Reinigungsoperation, beispielsweise einer Wasserdampfdestillation zur Entfernung von Verunreinigungen, und/oder einer Behandlung zur Verbesserung der Farbe, beispielsweise mit Aktivkohle, oder auch durch Umkristallisieren aus geeignetem Lösungsmittel gereinigt werden. Häufig ist jedoch eine solche Reinigung für die vorgesehene Anwendung nicht erforderlich. Die erfindungsgemäß hergestellten Verbindungen beziehungsweise Verbindungsgemische können auch versprüht werden um sie von restlichen, leicht verdampfenden Verbindungen beispielsweise Lösungsmitteln zu befreien und rieselfähige Pulver zu erhalten.

Während der Durchführung der weiter oben beschriebenen Reaktionen wird das Reaktionsgemisch zweckmäßig durch übliche Methoden, beispielsweise Rühren, Schütteln oder Umschaufeln, ständig bewegt und durchmischt.

Das erfindungsgemäße Verfahren ermöglicht es, quaternäre Ammoniumsalze von längerkettigen Carbonsäuren, die am Stickstoff einen längeren und drei kurzkettige Alkylreste tragen, mit guten Raum-Zeit-Ausbeuten ohne Entstehung abwasserbelastender Salze in üblichen unkomplizierten Reaktionsapparaten herzustellen, wobei überschüssig verwendete Reaktionspartner sowie bei der Reaktion entstehende, nützlich verwendbare Verbindungen wiedergewonnen werden können. Die erzeugten quaternären Ammoniumsalze der Formel (IV) beziehungsweise Mischungen aus Ammoniumsalzen der Foreml (IV) und Aminsalzen der Formel (V) können häufig ohne weitere Reinigungsoperationen verwendet werden.

Die Verbindungen der Formel (IV) sowie auch deren Mischungen mit Verbindungen der Formel (V) zeigen auf Textilien deutlich weichmachende Eigenschaften, so daß sie insbesondere in der Textilveredlung und Faserpräparation, in Wäscheweichspülmitteln oder in Waschpulvern eingesetzt werden können. Auf dem bevorzugten Anwendungsgebiet in Wäscheweichspülmitteln zeigen diese Verbindungen nicht die Nachteile des Standes der Technik beziehungsweise zeigen diese in deutlich verringertem Maße. Dies gilt insbesondere für ihre besonders gute Löslichkeit und Dispergierbarkeit, so daß sie sich auch in kaltem Spülwasser leicht verteilen lassen.

Bei der Bereitung von flüssigen Wäscheweichspülmitteln werden die in Rede stehenden Verbindungen zweckmäßig in Form ihrer konzentrierten Lösungen in niederen Alkanolen, vorzugsweise Isopropanol oder im Gemisch dieser Alkanole mit Wasser eingesetzt. Die erhaltenen Wäscheweichspülmittel enthalten in diesem Fall eine bestimmte Menge solcher niederen Alkanole (etwa 5 bis 30 Gew.-%), insbesondere weisen diese Alkanole 1 bis 5 C-Atome auf.

Ein weiterer Bestandteil dieser Wäscheweichspülmittel können übliche nicht-ionische Dispergatoren oder Emulgatoren auf Basis von Oxalkylaten sein, die zusätzlich dazu beitragen, daß das Wäscheweichspülmittel in kaltem Wasser gut dispergierbar ist. Geeignete nicht-ionische Dispergatoren sind beispielsweise Umsetzungsprodukte von jeweils etwa 2 bis 12 mol Ethylenoxid (EO) mit einem Alkylphenol wie Xylenol, aber auch mit einem Alkylphenol mit einem langen Alkylrest von 8 bis 10 C-Atomen oder mit einem Fettalkohol mit 8 bis 15 C-Atomen, insbesondere Umsetzungsprodukte von etwa 5 bis 8 mol EO mit 1 mol Alkylphenol oder mit 1 mol eines Fettalkohols oder eines Gemisches solcher Fettalkohole. Ebenfalls eingesetzt werden können Umsetzungsprodukte von jeweils 2 bis 12 mol Ethylenoxid mit Aminen, wie primären Alkylaminen oder sekundären Dialkylaminen. Die Menge dieser nicht-ionischen Verbindungen beträgt 3 bis 10 Gew.-% bei schwach konzentrierten Wäscheweichspülmitteln mit einem Gehalt der in Rede stehenden Verbindungen von 10 bis 30 Gew.-%. Höher konzentrierte Wäscheweichspülmittel mit einem Gehalt von 30 bis 70 Gew.-% enthalten vorzugsweise 5 bis 20 Gew.-% der nicht-ionischen Dispergatoren. Bei den verdünnten, in der Konzentration handelsüblichen Wäschweichspülmitteln mit einem Gehalt von weniger als 10 Gew.-%, insbesondere einem Gehalt von 1 bis 5 Gew.-%, ist die Zugabe von solchen Dispergatoren nicht erforderlich, sie kann aber beispielsweise bei 0,1 bis 3 Gew.-% liegen.

Die höher konzentrierten Wäschweichspülmittel enthalten darüber hinaus vorzugsweise noch 5 bis 30 Gew.-% eines Ethylenglykols, Propylenglykols, Polyethylenglykols, Polypropylenglykols oder die (C₁-C₄)Alkylether dieser Verbindungen. Von dieser Produktgruppe kommen nur solche Verbindungen in Frage, die flüssig sind. Diese Verbindungen zeigen in den Wäschweichspülmitteln eine lösungsvermittelnde Wirkung.

Nachfolgende Beispiele sollen die Erfindung näher erläutern:

### Beispiel 1

In einen Glasautoklaven von 1 dm³ Inhalt, der mit einem Rührer und einem Innenthermometer ausgestattet ist, werden 145,8 g (0,5 mol) einer Mischung von Alkyldimethylaminen mit folgender Kettenlängenverteilung der Alkylgruppe: C₁₂ = 1 Mol-%; C₁₄ = 3,2 Mol-%; C₁₆ = 35,8 Mol-%; C₁₈ = 58,9 Mol-%; C₂₀ = 1,1 Mol-% (Aminzahl 192,4; durchschnittliche Molmasse 291,6 g/mol) und 225,2 g (2,5 mol) Dimethylcarbonat gegeben, mit Stickstoff gespült und unter Rühren auf eine Innentemperatur von 140 °C erwärmt. Die Reaktionsmischung wird 6 Stunden unter Rühren auf 140 bis 142 °C gehalten, wobei der Druck im Autoklaven von 0,29 auf 0,43 MPa ansteigt. Nun wird auf 85 °C abgekühlt und auf normalen Atmosphärendruck entspannt. Nach ¹H-NMR-spektroskopischer Analyse hat sich das Amingemisch vollständig mit dem Dimethylcarbonat umgesetzt.
Es wurden nun 40 cm³ Isopropanol zugegeben und bei einer Autoklaven-Innentemperatur von 90 bis 100 °C mit Unterdruck bis 0,065 MPa, insgesamt 109 g flüchtiger Verbindungen (Dimethylcarbonat und Isopropanol), abdestilliert. Anschließend wird bei 100 °C innerhalb 25 min eine Mischung von 134,8 g (0,5 mol) eines Fettsäuregemisches mit folgender Kettenlängenverteilung: C₁₆ = 54 Mol-%; C₁₈ = 46 Mol-% (Säurezahl 208, durchschnittliche Molmasse 269,1 g/mol) und 15 cm³ Isopropanol unter Rühren zugetropft und dann während 50 min bei einer Temperatur von 100 °C bis zum Erreichen eines Unterdruckes von 0,03 MPa insgesamt 86 g flüchtige Verbindungen abdestilliert. Bei Raumtemperatur werden als fester Rückstand 315 g eines Gemisches von Verbindungen der Formel 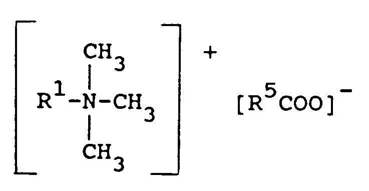
erhalten, worin bedeuten:
- R¹ =: Alkyl C₁₂; C₁₄; C₁₆; C₁₈ und C₂₀ in der oben angegebenen Kettenlängenverteilung und
- R⁵ =: Alkyl C₁₅ und C₁₇ im Molmengenverhältnis 5,4 : 4,6.

Nach gaschromatographischer Analyse enthält das Gemisch 4,4 Gew.-%, bezogen auf das Gemisch, restliches Isopropanol, was jedoch für die Verwendung des erzeugten Produktes, beispielsweise als Wäscheweichspülmittel, nicht stört.

### Beispiel 2

In dieselbe Apparatur, wie in Beispiel 1 beschrieben, werden 122,6 g (0,5 mol) Tetradecyldimethylamin (Aminzahl 228,9) und 45,0 g (0,5 mol) Dimethylcarbonat gegeben, mit Stickstoff gespült und 10 Stunden unter Rühren auf eine Innentemperatur von 132 bis 142 °C bei einem Druck von 0,18 bis 0,2 MPa erwärmt, dann auf 100 °C abgekühlt und entspannt. Nach ¹H-NMR-spektroskopischer Analyse hat sich mit einem Umsatz von 80 % Tetradecyl-trimethyl-ammonium-methylcarbonat gebildet. Nun werden unter fortgesetztem Rühren nacheinander mit einer Kolbenpumpe während 5 min 16,8 g Methanol und während 20 min 148,9 g (0,5 mol) Isostearinsäure (Säurezahl 188,4) zugegeben und anschließend bei 80 bis 100 °C bis zum Erreichen eines Unterdruckes von 0,003 MPa die flüchtigen Bestandteile (vorwiegend Methanol und Dimethylcarbonat) abdestilliert. Es werden 265 g eines bei Raumtemperatur festen Gemisches erhalten, das nach ¹H-NMR-spektroskopischer Analyse zu 80 Gew.-% aus Tetradecyltrimethyl-ammonium-isostearat und zu 20 Gew.-% aus Tetradecyl-dimethyl-ammonium-isostearat (beide Gewichtsprozent-Angaben bezogen auf das Gemisch) besteht.

### Beispiel 3

In dieselbe Apparatur, wie in Beispiel 1 beschrieben, werden 116,1 g (0,4 mol) einer Mischung von Alkyldimethylaminen derselben Zusammensetzung wie in Beispiel 1 und 180,2 g (2,0 mol) Dimethylcarbonat gegeben, mit Stickstoff gespült und 3 Stunden unter Rühren auf eine Innentemperatur von 135 bis 137 °C bei einem Druck von 0,22 bis 0,26 MPa erwärmt. Nun werden innerhalb 1 Stunde bei Temperaturen von 137 bis 110 °C 68 g flüchtige Verbindungen (überwiegend Dimethylcarbonat) abdestilliert und anschließend bei 90 °C während 35 min eine flüssige Mischung von 129,6 g (0,48 mol) des in Beispiel 1 beschriebenen Fettsäuregemisches (C₁₆ und C₁₈) und 85 cm³ Isopropanol zudosiert. Danach werden bei Temperaturen von 90 bis 100 °C bis zum Erreichen eines Unterdruckes von 0,065 MPa während 40 min insgesamt 96 g flüchtige Verbindungen (vorwiegend Isopropanol, Methanol und Dimethylcarbonat) abdestilliert. Es werden 245 g eines bei Raumtemperatur festen Gemisches erhalten, das nach ¹H-NMR-spektroskopischer Analyse zu 75 Gew.-% aus Verbindungen der Formel 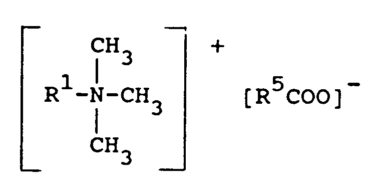
zu 8 Gew.-% aus Verbindungen der Formel 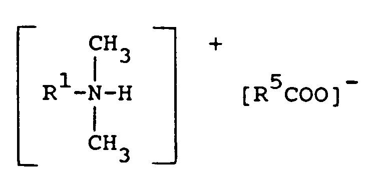
und zu 17 Gew.-% aus Verbindungen der Formel

R⁵COOH

besteht, wobei sich die Gewichtsprozent-Angaben auf das gesamte erhaltene feste Gemisch beziehen und in den oben genannten Formeln bedeuten:
- R¹ =: Alkyl C₁₂; C₁₄; C₁₆; C₁₈ und C₂₀ in der in Beispiel 1 angegebenen Kettenlängen-Verteilung und
- R⁵ =: Alkyl C₁₅ und C₁₇ im Molmengenverhältnis 5,4 : 4,6

### Beispiel 4

In dieselbe Apparatur, wie im Beispiel 1 beschrieben, werden 112,6 g (0,388 mol) einer Mischung von Alkyldimethylaminen derselben Zusammensetzung wie in Beispiel 1 und 86,2 g (0,957 mol) Dimethylcarbonat gegeben, mit Stickstoff gespült und 4 Stunden unter Rühren auf eine Innentemperatur von 135 bis 142 °C, bei einem Druck von 0,22 bis 0,38 MPa, erwärmt. Nun wird auf 80 °C abgekühlt, auf normalen Atmosphärendruck entspannt und nacheinander 36 g Isopropanol und 18 g (1 mol) Wasser unter Rühren zugetropft. Die Innentemperatur wird auf 75 bis 80 °C gehalten und nach der Wasserzugabe während 25 min unter fortgesetztem Rühren 104,7 g (0,388 mol) des auf 80 °C vorgewärmten, in Beispiel 1 beschriebenen Fettsäuregemisches (C₁₆ und C₁₈) tropfenweise zugegeben. Anschließend werden bei 90 °C bis zum Erreichen eines Unterdruckes von 0,067 MPa insgesamt 85,7 g flüchtige Verbindungen abdestilliert. Es werden 244 g eines bei Raumtemperatur festen Gemisches erhalten, das nach ¹H-NMR-spektroskopischer Analyse 90 Gew.-% Verbindungen der Formel 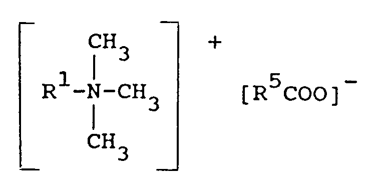
worin bedeuten:
- R¹ =: Alkyl C₁₂; C₁₄; C₁₆; C₁₈ und C₂₀ in der in Beispiel 1 angegebenen Kettenlängen-Verteilung und
- R⁵ =: Alkyl C₁₅ und C₁₇ im Molmengenverhältnis 5,4 : 4,6
ferner 4,5 Gew.-% Wasser und 5,5 Gew.-% Isopropanol enthält, wobei sich alle Gewichtsprozent-Angaben auf das gesamte erhaltene feste Gemisch beziehen.

## Patentansprüche

1. Verfahren zur Herstellung von quaternären Ammoniumsalzen langkettiger aliphatischer Carbonsäuren, die am Stickstoffatom drei kurzkettige und einen langkettigen aliphatischen Kohlenwasserstoffrest tragen, dadurch gekennzeichnet, daß man
a) 1 mol von mindestens einer Verbindung der Formel 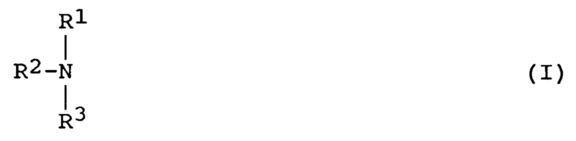 in der bedeuten
R¹ einen geradkettigen oder verzweigten, aliphatischen Kohlenwasserstoffrest mit 8 bis 22 C-Atomen, der 1 bis 3 C=C-Doppelbindungen und/oder 1 bis 3 OH-Gruppen enthalten kann, und R² und R³, gleich oder verschieden, je einen Alkylrest mit 1 bis 4 C-Atomen,
mit 0,5 bis 6 mol einer Verbindung der Formel
R⁴OCOOCH₃ (II)
in der R⁴ Methyl- oder Ethyl- bedeutet,
bei 100 bis 200 °C unter einem Druck von 0,095 bis 1,5 MPa umsetzt,
b) von der im Schritt a) erhaltenen Reaktionsmischung die Hauptmenge der nicht umgesetzten Verbindung der Formel (II) gegebenenfalls abtrennt,
c) der im Schritt a) oder, sofern ausgeführt, im Schritt b) erhaltenen Reaktionsmischung je 1 mol der eingesetzten Verbindung der Formel (I) 1 bis 2 mol von mindestens einer Carbonsäure der Formel
R⁵COOH (III)
worin R⁵ einen geradkettigen oder verzweigten, aliphatischen Kohlenwasserstoffrest mit 8 bis 40 C-Atomen, der 1 oder 2 COOH-Gruppen, 1 bis 3 C=C-Doppelbindungen, 1 bis 3 CHOH-Gruppen sowie einen Ring enthalten kann,
bedeutet, bei einer Temperatur von 40 bis 140 °C zusetzt und diese Temperatur aufrechterhält, bis keine CO₂-Entwicklung mehr auftritt, und
d) die restliche Menge an CO₂ und den im Schritt c) ebenfalls gebildeten Alkohol R⁴OH von der im Schritt c) erhaltenen Reaktionsmischung abdestilliert, wobei das gewünschte quaternäre Ammoniumsalz der Formel 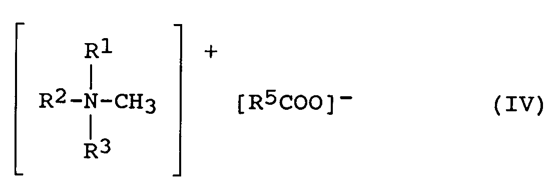 erhalten wird.

2. Verfahren nach Anspruch 1, dadurch gekennzeichnet, daß nach Beendigung des Schrittes a) oder, sofern ausgeführt, nach Beendigung des Schrittes b) der Reaktionsmischung je 1 mol der in dieser Mischung vorliegenden Verbindung der Formel (II) 1 bis 10 mol Wasser zugesetzt werden.

3. Verfahren nach Anspruch 1 oder 2, dadurch gekennzeichnet, daß R¹ einen geradkettigen oder verzweigten, aliphatischen Kohlenwasserstoffrest mit 12 bis 20 C-Atomen, der 1 bis 3 C=C-Doppelbindungen und/oder 1 bis 3 OH-Gruppen enthalten kann, bedeutet.

4. Verfahren nach einem oder mehreren der Ansprüche 1 bis 3, dadurch gekennzeichnet, daß R⁵ einen geradkettigen oder verzweigten, aliphatischen Kohlenwasserstoffrest mit 8 bis 22 C-Atomen, der 1 bis 3 C=C-Doppelbindungen und/oder 1 bis 3 OH-Gruppen enthalten kann, bedeutet.

5. Verfahren nach einem oder mehreren der Ansprüche 1 bis 4, dadurch gekennzeichnet, daß nach Zusatz der Verbindung der Formel (III) im Schritt c) die Temperatur der Reaktionsmischung auf 60 bis 120 °C eingestellt wird.

## Claims

1. A process for the production of quaternary ammonium salts of long-chain aliphatic carboxylic acids carrying three short-chain and one long-chain aliphatic hydrocarbon radicals on the nitrogen atom, characterized by
a) reacting 1 mole of at least one compound of the formula 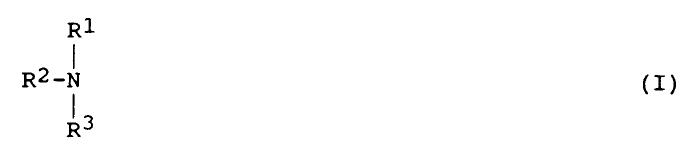 in which R¹ denotes a linear or branched aliphatic hydrocarbon radical which has 8 to 22 carbon atoms and can contain 1 to 3 C=C double bonds and/or 1 to 3 OH groups, and R² und R³ are identical or different and denote in each case an alkyl radical having 1 to 4 carbon atoms,
with 0,5 to 6 moles of a compound of the formula
R⁴OCOOCH₃ (II)
in which R⁴ denotes methyl or ethyl,
at 100 to 200 °C and under a pressure of 0,095 to 1,5 MPa,
b) removing the bulk of the unreacted compound of the formula (II) from the reaction mixture obtained in step a), if necessary,
c) adding to the reaction mixture obtained in step a) or in step b) if this step has been carried out, 1 to 2 moles, per mole of compound of the formula (I) employed, of at least one carboxylic acid of the formula
R⁵COOH (III)
in which R⁵ denotes a linear or branched, aliphatic hydrocarbon radical which has 8 to 40 carbon atoms and can contain 1 or 2 COOH groups, 1 to 3 C=C douple bonds, 1 to 3 CHOH groups and a ring,
at a temperature of 40 to 140 °C, and maintaining this temperature until no more CO₂ formation occurs, and
d) distilling off the residual amount of CO₂ and the alcohol R⁴OH formed as well in step c), from the reaction mixture obtained in step c), obtaining thereby the desired quaternary ammonium salt of the formula

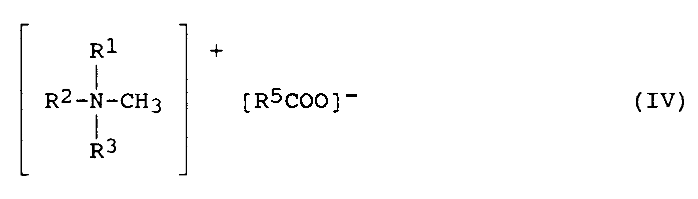

2. The process as claimed in claim 1, wherein after the completion of step a) or step b) if this step has been carried out, 1 to 10 moles of water are added to the reaction mixture per mole of the compound of the formula (II) in this mixture.

3. The process as claimed in claim 1 or 2, wherein R¹ denotes a linear or branched aliphatic hydrocarbon radical which has 12 to 20 carbon atoms and can contain 1 to 3 C=C double bonds and/or 1 to 3 OH groups.

4. The process as claimed in claim 1 to 3, wherein R⁵ denotes a linear or branched aliphatic hydrocarbon radical which has 8 to 22 carbon atoms and can contain 1 to 3 C=C double bonds and/or 1 to 3 OH groups.

5. The process as claimed in one or more of the claims 1 to 4, wherein after the addition of the compound of the formula (III) in step c), the temperature of the reaction mixture is adjusted to 60 to 120 °C.

## Revendications

1. Procédé pour préparer des sels d'ammonium quaternaires d'acides carboxyliques aliphatiques à longue chaîne, portant sur l'atome d'azote trois radicaux hydrocarbonés aliphatiques à courte chaîne et un radical hydrocarboné aliphatique à longue chaîne, caractérisé en ce que :
a) on fait réagir 1 mole d'au moins un composé de formule 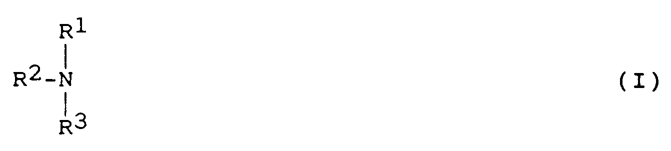 dans laquelle R¹ est un radical hydrocarboné aliphatique à chaîne droite ou ramifiée ayant de 8 à 22 atomes de carbone et pouvant porter de 1 à 3 doubles liaisons C=C et/ou 1 à 3 groupes OH, et R² et R³, qui sont identiques ou différents, représentent chacun un radical alkyle ayant de 1 à 4 atomes de carbone,
avec de 0,5 à 6 moles d'un composé de formule
R⁴OCOOCH₃ (II)
dans laquelle R⁴ est le radical méthyle ou éthyle,
à une température de 100 à 200°C et sous une pression de 0,095 à 1,5 MPa,
b) on sépare éventuellement du mélange réactionnel obtenu dans l'étape a) la majorité du composé de formule (II) n'ayant pas réagi,
c) à une température de 40 à 140°C, on ajoute au mélange réactionnel obtenu dans l'étape a) ou encore, si elle est mise en oeuvre, dans l'étape b), et par mole du composé utilisé de formule (I), 1 à 2 moles d'au moins un acide carboxylique de formule
R⁵COOH (III)
dans laquelle R⁵ est un radical hydrocarboné aliphatique à chaîne droite ou ramifiée ayant de 8 à 40 atomes de carbone et pouvant comporter un ou deux groupes COOH, 1 à 3 doubles liaisons C=C, 1 à 3 groupes CHOH, ainsi qu'un cycle,
et on maintient cette température jusqu'à arrêt du dégagement de CO₂, et
d) on chasse par distillation, du mélange réactionnel obtenu dans l'étape c), la quantité résiduelle de CO₂ et l'alcool R⁴OH qui lui aussi est formé au cours de l'étape c), ce qui permet d'obtenir le sel d'ammonium quaternaire recherché :

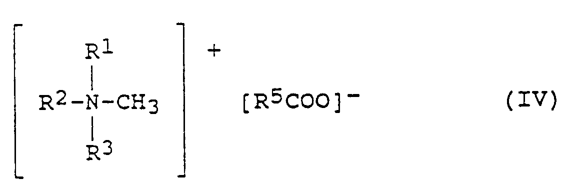

2. Procédé selon la revendication 1, caractérisé en ce que, après la fin de l'étape a) ou encore, si elle est effectuée, après la fin de l'étape b), on ajoute au mélange réactionnel 1 à 10 moles d'eau par mole du composé de formule (II) présent dans ce mélange.

3. Procédé selon la revendication 1 ou 2, caractérisé en ce que R¹ est un radical hydrocarboné aliphatique à chaîne droite ou ramifiée ayant de 12 à 20 atomes de carbone et pouvant contenir 1 à 3 doubles liaisons C=C et/ou 1 à 3 groupes OH.

4. Procédé selon l'une ou plusieurs des revendications 1 à 3, caractérisé en ce que R⁵ est un radical hydrocarboné aliphatique à chaîne droite ou ramifiée ayant de 8 à 22 atomes de carbone, et pouvant contenir 1 à 3 doubles liaisons C=C et/ou 1 à 3 groupes OH.

5. Procédé selon l'une ou plusieurs des revendications 1 à 4, caractérisé en ce que, après addition du composé de formule (III) dans l'étape c), on ajuste le mélange réactionnel à une température de 60 à 120°C.
